# EUROPÄISCHE PATENTSCHRIFT

(11) **EP 2 298 170 B1**
(45) Veröffentlichungstag und Bekanntmachung des Hinweises auf die Patenterteilung: **02.04.2014**
(21) Anmeldenummer: 10182157.7
(22) Anmeldetag: 15.09.2004
(51) Int. Cl.: A61B 5/15, A61B 5/00

(54) **Testgerät zur Untersuchung von Körperflüssigkeiten**
Test device for analyzing body fluids
Dispositif d'essai pour examiner des liquides organiques

(30) Priorität: 19.09.2003 DE 10343896
(43) Veröffentlichungstag der Anmeldung: 23.03.2011
(62) Teilanmeldung aus: 04765203.7
(73) Patentinhaber: F. Hoffmann-La Roche AG, 4070 Basel (CH); Roche Diagnostics GmbH, 68305 Mannheim (DE)
(72) Erfinder: Hoenes, Joachim, Dr., 64673, Zwingenberg (DE); List, Hans, 64754, Hesseneck-Kailbach (DE); Miltner, Karl, 67227, Frankenthal (DE); Schmid, Wilfried, Dr., 68165, Mannheim (DE)
(74) Vertreter: Pfiz, Thomas

(56) Entgegenhaltungen:
- EP-A- 0 299 517
- WO-A-02/100274
- DE-A1- 19 819 407
- US-A- 5 228 972

## Beschreibung

Die Erfindung betrifft ein Testgerät zur Untersuchung von Körperflüssigkeiten wie Blut oder Urin, mit einem vorzugsweise in einer Bandkassette unter Bandvorlauf aus einem Vorratsraum in einen Abfallraum transportierbaren Testband, welches eine Vielzahl von an einer Aufnahmeposition mit Körperflüssigkeit beaufschlagbaren Testabschnitten aufweist, und einer Messeinheit zum Nachweis eines lnhaltsstoffes der Körperflüssigkeit auf einem aktiven Testabschnitt.

Für Diabetiker sind regelmäßige Blutzuckerkontrollen unerlässlich, um Therapie, Ernährung und Lebensrhythmus auf die jeweiligen Erfordernisse einstellen zu können. Zur Selbstkontrolle sind gleichsam als Minilabore arbeitende Handgeräte am Markt, mit denen auch von Laien die erforderlichen Schritte einfach und schnell vorgenommen werden können. Dabei werden disposible Teststreifen bereitgehalten, um nach Beaufschlagung mit Kapillarblut einen geräteinternen Nachweis beispielsweise durch eine optische Messeinheit zu ermöglichen. Die Magazinierung und Verarbeitung der Teststreifen beansprucht allerdings viel Bauraum und macht aufwendige Antriebe erforderlich. In den zum Zeitpunkt dieser Anmeldung noch unveröffentlichten EP-Anmeldungen Nr. 02026242.4 und 02028894.0 wird deshalb vorgeschlagen, anstelle einzelner Teststreifen ein aufgewickeltes Testband zu verwenden, auf dem eine Vielzahl von mit einer geeigneten Testchemie versehenen Testabschnitten fortlaufend angeordnet sind. Die Körperflüssigkeit wird auf einem durch Bandvorlauf in eine aktive Position gebrachten Testabschnitt appliziert und analysiert. Einzelheiten zur Blutgewinnung sowie zu bekannten Testmedien und Nachweissystemen insbesondere für Blutglucose sind dort angegeben, worauf hier Bezug genommen und der diesbezügliche Inhalt in diese Anmeldung aufgenommen wird.

Ausgehend hiervon liegt der Erfindung die Aufgabe zugrunde, die im Stand der Technik aufgetretenen Nachteile zu vermeiden und ein Gerät der eingangs genannten Art zu verbessern und zu vereinfachen, so dass ein zuverlässiger Messablauf erreichbar ist.

US 5,228,972 und EP 0 299 517 A2 beschreiben die Merkmale gemäß des Oberbegriffs des Anspruchs 1.

Zur Lösung dieser Aufgabe wird die im Patentansprüche 1 und 14 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den für die sukzessive Positionierung der Testabschnitte ohnehin erforderlichen Bandtransport für Zusatzfunktionen zu nutzen. Dementsprechend wird erfindungsgemäß vorgeschlagen, dass das Testband mindestens einen durch Bandvorlauf in eine Funktionsstellung bringbaren Funktionsabschnitt für eine Gerätehilfsfunktion zusätzlich zu den Testabschnitten aufweist. Auf diese Weise können ansonsten ungenutzte Bandabschnitte außerhalb der Testbereiche genutzt werden, um außerhalb der eigentlichen Messung beim Bandvorlauf automatisch Zusatzfunktionen zu bewirken.

Gemäß einer vorteilhaften Ausführung wirken der/die Funktionsabschnitt/e in der Funktionsstellung mit der Messeinheit zusammen. Grundsätzlich ist es aber auch möglich, dass der Funktionsabschnitt durch eine gesonderte Erfassungseinheit beispielsweise optisch, elektrisch oder magnetisch abgetastet wird.

Vorteilhafterweise besitzt das Kalibrierfeld eine definierte optische Eigenschaft für eine Kalibration einer optischen Messeinheit. Zur Verbesserung der Messgenauigkeit wird vorgeschlagen, dass der Funktionsabschnitt ein insbesondere weißes, schwarzes, graues, farbiges oder transparentes Kalibrierfeld zur insbesondere optischen Kalibration der Messeinheit aufweist. Für eine Dunkelkalibration der Messeinheit kann ein transparenter Bereich einer Trägerfolie des Testbandes als Funktionsabschnitt ausgebildet sein.

Eine weitere Verbesserung wird durch einen Komparator zum Vergleich eines an einem unbenutzten Testabschnitt oder Funktionsabschnitt erfassten Messwertes mit einem Sollwert erreicht. Ein solcher Komparator kann durch eine Vergleichsschaltung oder eine Software auf einem Auswerteprozessor gebildet sein.

Zur selbsttätigen Reinigung beim Bandvorlauf ist es von Vorteil, wenn der Funktionsabschnitt ein beim Bandvorlauf mit der Messeinheit in Kontakt kommendes Reinigungsfeld aufweist.

Eine Schutzfunktion lässt sich dadurch erreichen, dass der Funktionsabschnitt ein zum Schutz der Messeinheit ausgebildetes, insbesondere elastisch dehnbares Schutzfeld aufweist.

Günstig ist es auch, wenn der Funktionsabschnitt ein Sensorfeld zur Erfassung von Umgebungsparametern wie Temperatur oder Luftfeuchtigkeit aufweist.

Zur Steuerung des Messablaufs der Messeinheit ist es vorteilhaft, wenn der Funktionsabschnitt ein insbesondere durch eine Metallfolie gebildetes Steuerfeld aufweist.

Für eine Benutzerinformation kann der Funktionsabschnitt ein in Klarschrift direkt ablesbares Informationsfeld aufweisen. In vorteilhafter Ausgestaltung ist es vorgesehen, dass die Informationsfelder zwischen den Testabschnitten angeordnet sind und eine die Anzahl der noch verfügbaren Testabschnitte anzeigende Reststandnummerierung aufweisen.

Eine weitere Verbesserung der Funktionalität und insbesondere der Hygiene wird dadurch erreich, dass der Funktionsabschnitt ein zur Entsorgung von Körperflüssigkeit ausgebildetes Saugfeld aufweist. In diesem Zusammenhang ist es auch denkbar, dass der Funktionsabschnitt ein beispielsweise durch Feuchtigkeit aktivierbares Konservierungsmittel enthält.

Vorteilhafterweise sind der bzw. die Funktionsabschnitte als Multifunktionsfeld für eine Kombination von mehreren Gerätehilfsfunktionen, insbesondere für eine Kalibration der Messeinheit sowie eine Steuerung des Messablaufs ausgebildet.

Vorteilhafterweise sind in Bandlaufrichtung gesehen Funktionsabschnitte abwechselnd zu den Testabschnitten angeordnet. Dabei lässt sich mittels der an einem Funktionsabschnitt kalibrierten Messeinheit ein Leerwert an einem unbenutzten Testabschnitt kontrollieren.

Vorteilhafterweise ist das Testband von einer Vorratsspule in dem Vorratsraum abwickelbar, über Führungsmittel im Bereich der Aufnahmeposition führbar und auf eine Aufwickelspule in dem Abfallraum aufwickelbar.

Um eine geeignete Testchemie vorzuhalten, ist es von Vorteil, wenn die Testabschnitte durch eine auf einem kontinuierlichen Trägerband abschnittsweise aufgebrachte Reagenzbeschichtung gebildet sind.

Eine weitere Verbesserung speziell der Handhabung wird dadurch erreicht, dass das Testband in einer Bandkassette in ein Gehäuse eines tragbaren Geräts einsetzbar ist.

Eine entsprechende Bandkassette als Verbrauchsmaterial zum Einsatz in einem Testgerät ist ebenfalls Gegenstand der Erfindung.

Im Folgenden wird die Erfindung anhand eines in der Zeichnung in schematischer Weise dargestellten Ausführungsbeispiels näher erläutert. Es zeigen
- Fig. 1: ein tragbares Blutzuckermessgerät für Diabetiker mit einem Test- band in vereinfachter geschnittener Darstellung;
- Fig. 2: das Testband mit Testabschnitten und zusätzlichen Funktionsab- schnitten in abgebrochener perspektivischer Ansicht.

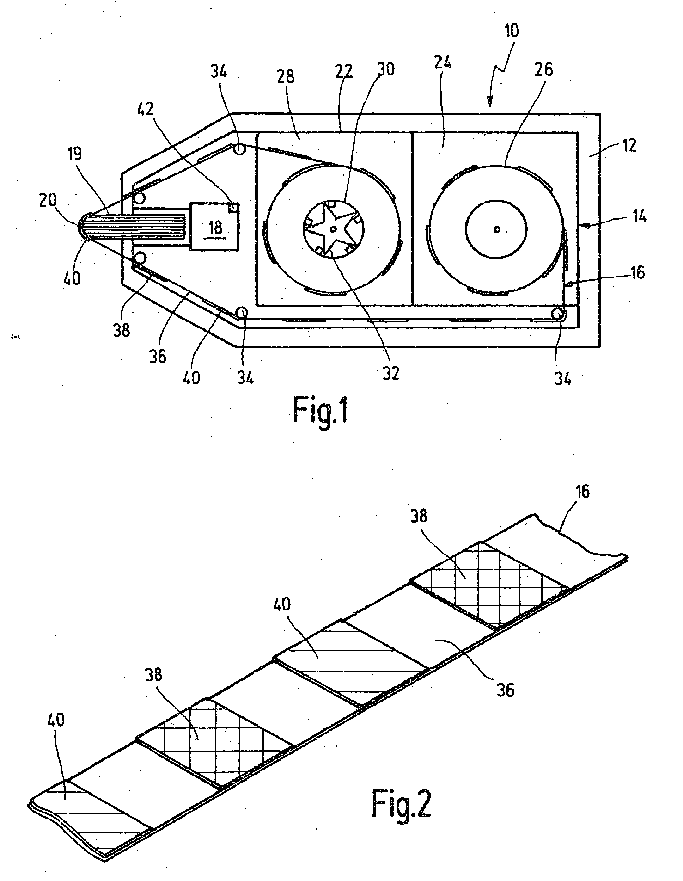

Das in Fig. 1 dargestellte Blutzuckermessgerät 10 umfasst ein Gerätegehäuse 12, eine darin eingesetzte Bandkassette 14 mit Testband 16 und eine optische Messeinheit 18 zur Analyse von auf dem Testband applizierfem Blut. Die Messeinheit 18 ist mit einer Führungsspitze 19 im Bereich einer Blutaufnahmeposition 20 zum gezielten Auftragen eines Bluttropfens auf das Testband gekoppelt. Das generelle Geräteprinzip ist in der EP-Anmeldung Nr. 02026242.4 detailliert beschrieben, worauf Bezug genommen und der diesbezügliche Inhalt hier aufgenommen wird.

Die Bandkassette 14 weist ein Kassettengehäuse 22 mit einer Vorratskammer 24 für eine Vorratsspule 26 und einer Speicherkammer 28 für eine Aufwickelspule 30 auf. Ein Spulenantrieb 32 ermöglicht das abschnittsweise Vorspulen des Testbandes 16 von der Vorratsspule 26 auf die Aufwickelspule 30. Zwischen den Spulen 26, 30 ist der freilaufende Teil des Testbandes 16 über Umlenkrollen 34 und über die Führungsspitze 19 geführt.

Wie auch aus Fig. 2 ersichtlich, weist das Testband 16 eine kontinuierliche Trägerfolie 36 auf, die mit Testabschnitten 38 für Analysezwecke und zusätzlichen Funktionsabschnitten 40 für Gerätehilfsfunktionen versehen ist. Die Trägerfolie 36 besteht aus einer etwa 20 µm dünnen Polymerfolie beispielsweise aus Polyester.

Im Bereich der Testabschnitte 38 sind Reagenzfelder beispielsweise durch Klebe- oder Etikettierverfahren unter Verwendung vorgefertigter Testelemente oder durch direkte Beschichtungsverfahren, insbesondere Druckverfahren aufgebracht. Die Reagenzfelder enthalten Trockenchemikalien, die auf den Analyten (Glucose) in der applizierten Blutflüssigkeit durch eine Farbänderung ansprechen. Der Nachweis dieser Reaktion erfolgt durch reflexionsphotometrische Messung mittel der Messeinheit 18. Dabei ist die Messeinheit 18 über die als Lichtleiter ausgebildete Führungsspitze 19 und durch die transparente Trägerfolie 36 hindurch an einen in der Aufnahmeposition 20 befindlichen momentan aktiven Testbandabschnitt 38 optisch angekoppelt. Durch entsprechenden Bandvorlauf können die Testabschnitte 38 sukzessive zum Einsatz gebracht werden. Auf diese Weise lassen sich eine Vielzahl von automatisch ablaufenden Tests für eine Patienten-Selbstkontrolle durchführen, ohne dass Verbrauchsmittel ausgetauscht werden müssten.

Beim Bandtransport gelangen auch die Funktionsabschnitte 40 in ihre jeweilige Funktionsstellung. Dabei kann wie in Fig. 1 dargestellt ein Zusammenwirken mit der Messeinheit 18 vorgesehen sein. Alternativ oder ergänzend erfüllen die Funktionsabschnitte 38 Zusatzfunktionen gegenüber anderen Gerätekomponenten oder dem Benutzer.

In der gezeigten Ausführungsform sind die Funktionsabschnitte 40 in Bandlaufrichtung abwechselnd zu den Testabschnitten 38 und auf derselben Bandseite durch Aufdrucken oder Aufkleben oder mit anderen geeigneten Maßnahmen auf der Trägerfolie 36 aufgebracht. Abhängig von der gewünschten Funktion sind auch andere Anordnungen denkbar, beispielsweise nur am Anfang des Testbandes 16 oder auf der von der Blutapplikationsseite abgewandten Bandrückseite.

Für eine Kalibration der optischen Messeinheit 18 können die Funktionsabschnitte 38 durch ein in seinen optischen Eigenschaften definiertes Kalibrierfeld gebildet sein. Hierfür können weiße, schwarze, graue oder farbige Felder verwendet werden. Durch die Kalibration kann eine Alterung oder sonstige Veränderung während der Gebrauchszeit kompensiert werden, so dass eine gleichbleibende Messgenauigkeit gewährleistet ist. Farbige Felder können zur Wellenlängenkontrolle und ggf. zur Driftkorrektur beispielsweise bei Temperaturschwankungen benutzt werden. Zur Dunkelkalibrierung kann bereits ein Bereich der transparenten Trägerfolie 36, welche praktisch kein Licht remittiert, genügen. Die Sollwerte der Kalibrierfelder werden zweckmäßigerweise chargenspezifisch in das Gerät 10 eingespeist, z. B. über ein Funktionsfeld 40 als Kodier- bzw. Speichermedium, welches mit einer nicht gezeigten Auswerteeinheit zusammenwirkt.

Die Kalibration der Messeinheit 18 erfolgt über einen Komparator 42 durch Vergleich mit einem im Gerät hinterlegten Sollwert oder Kennfeld. Die Justierung der Messeinheit 18 ermöglicht auch eine sehr genaue Kontrolle des Leerwertes an einem unbenutzten Testabschnitt 38. Damit kann eine Verfärbung oder sonstige Veränderung, die auf Unbrauchbarkeit hindeutet, sehr empfindlich erkannt werden.

Eine Reinigungsfunktion lässt sich dadurch erreichen, dass ein Funktionsfeld 40 aus einem weichen Material wie Filz oder Papier auf der der Messeinheit 18 zugewandten Rückseite des Trägerbandes 36 aufgebracht ist. Beim Bandvorlauf gleitet dieses Reinigungsfeld über das Messfenster und entfernt dadurch automatisch Schmutz und Staub, ohne dass zusätzliche Geräte-oder Benutzereingriffe erforderlich wären.

Im Ruhezustand wird die Optik der Messeinheit 18 von dem dünnen transparenten Trägerband 36 kaum geschützt. Um hier Abhilfe zu schaffen, kann sich in der Ruheposition ein als Schutzfeld geeigneter Funktionsabschnitt 40 vor der Optik befinden, um so eine bessere Abschirmung gegen Umgebungseinflüsse zu erreichen. Beispielsweise ist hierfür ein elastisches Flachmaterialstück geeignet. Ein solches Schutzfeld lässt sich auch zuverlässiger führen, so dass ein seitliches Abgleiten von der Führungsspitze 19 verhindert werden kann.

Eine weitere Anwendung der Funktionsabschnitte 40 besteht darin, dass temperaturabhängig sich verfärbende Felder als Sensoren für die aktuelle Temperatur oder die Temperatur während der Lagerung dienen und ggf. eine Temperaturüberschreibung anzeigen. Ein Feuchte-sensitives Funktionsfeld könnte ebenfalls vor ungeeigneten Bedingungen warnen.

Eine Metallfolie als Funktionsabschnitt 40 auf dem Trägerband 36 und ein Näherungsschalter im Gerät 10 können Start- und Stopp-Signale auslösen oder eine Geräteklappe steuern. Metallfolien unterschiedlichen Widerstands könnten elektrisch "gelesen" werden. Ein magnetisierbares Folienstück könnte gelesen und auch beschrieben werden.

Weiterhin ist es möglich, dass die Funktionsabschnitte durch den Benutzer direkt ablesbare Informationen wie z. B. die Anzahl der noch verfügbaren Testabschnitte 38 enthalten. Dadurch kann der Benutzer auch an einer aus dem Gerät 10 entnommenen Kassette 14 die Reststandsnummerierung erkennen. Ein farbiges Warnfeld vor den letzten Testabschnitten 38 könnte den Benutzer auf das baldige Bandende aufmerksam machen.

Eine weitere Zusatzfunktion besteht darin, dass ein saugfähiges Funktionsfeld 40 einen Blutprobenüberschuss aufnimmt, so dass dieser in der Abfallkammer 28 hygienisch trocknen kann. Ein Funktionsfeld mit einem Konservierungsmittel könnte bei Bedarf in der Abfallkammer 28 die Hygiene weiter verbessern.

Möglich ist es auch, dass ein Funktionsfeld 40 mehrere Hilfsfunktionen gleichzeitig übernimmt. Beispielweise kann ein etwas dickeres weißes Papierfeld für Kalibrierung, Reinigung und Schutz verwendet werden. Der Anfang dieses Feldes reinigt nach der Messung. Der Mittelteil liegt während des Nichtgebrauchs vor der Optik und schützt diese. Auf dem Mittelteil ist für den Kunden sichtbar die laufende Testzahl aufgedruckt. Der Endteil wird vor der neuen Messung zum Kalibrieren der Optik benutzt. In der Abfallkammer 28 wird ein Überschuss Blut aufgenommen und die Hygiene verbessert.

Zusammenfassend ist folgendes festzuhalten: Die Erfindung betrifft ein Testgerät zur Untersuchung von Körperflüssigkeiten wie Blut oder Urin, mit einem aus einem Vorratsbereich 24 in einen Abfallbereich 28 transportierbaren aufgewickelten Testband 16, welches eine Vielzahl von an einer Aufnahmeposition 20 mit Körperflüssigkeit beaufschlagbaren Testabschnitten aufweist, und einer Messeinheit 18 zum Nachweis eines lnhaltsstoffes der Körperflüssigkeit auf einem aktiven Testabschnitt 38. Für die Ausführung von erätehilfsfunktionen wird vorgeschlagen, dass das Testband 16 einen oder mehrere unter Bandvorlauf in eine Funktionsstellung bringbare Funktionsabschnitte 40 zusätzlich zu den Testabschnitten aufweist.

## Patentansprüche

1. Testgerät zur Untersuchung von Körpertlüssigkeiten wie Blut oder Urin, mit einem vorzugsweise in einer Bandkassette (14) unter Bandvorlauf aus einem Vorratsraum (24) in einen Abfallraum (28) transportierbaren Testband (16), welches eine Vielzahl von an einer Aufnahmeposition (20) mit Körperflüssigkeit beaufschlagbaren Testabschnitten aufweist, wobei die Testabschnitte (38) durch Reagenzfelder gebildet sind, die auf einen Inhaltsstoff der Körperflüssigkeit durch eine Farbänderung ansprechen, und einer optischen Messeinheit (18) zum Nachweis des Inhaltsstoffes der Körperflüssigkeit auf einem aktiven Testabschnitt (38), wobei das Testband (16) mehrere durch Bandvorlauf in eine Funktionsstellung bringbare Funktionsabschnitte (40) für eine Gerätehilfsfunktion zusätzlich zu den Testabschnitten aufweist, **dadurch gekennzeichnet, dass** abwechseln zu den Testabschnitten Funktionsabschnitte (40) angeordnet sind und ein weißes, schwarzes, graues. farbiges oder transparentes Kalibrierfeld zur Kalibration der Messeinheit (18) und/oder ein Steuerfeld zur Steuerung des Messablaufs der Messeinheit (18) aufweisen.

2. Testgerät nach Anspruch 1, **dadurch gekennzeichnet, dass** der Funktionsabschnitt (40) in der Funktionsstellung mit der Messeinheit (18) zusammenwirkt.

3. Testgerät nach Anspruch 1 oder 2, **dadurch gekennzeichnet, dass** das Kalibrierfeld eine definierte optische Eigenschaft für eine Kalibration einer optischen Messeintteit (18) aufweist

4. Testgerät nach einem der Ansprüche 1 bis 3, **dadurch gekennzeichnet, dass** ein transparenter Bereich einer Trägerfolie (36) des Testbandes (16) als Funktionsabschnitt zur Dunkelkalibration der Messeinheit (18) ausgebildet ist.

5. Testgerät nach einem der Ansprüche 1 bis 4, **gekennzeichnet durch** einen Komparator (42) zum Vergleich eines an einem unbenutzten Testabschnitt (38) oder Funktionsabschnitt (40) erfassten Messwertes mit einem vorzugsweise geräteseitig hinterlegten oder in das Gerät eingespeisten Sollwert.

6. Testgerät nach einem der Ansprüche 1 bis 5, **dadurch gekennzeichnet, dass** der Funktionsabschnitt (40) ein durch eine Metallfolie gebildetes Steuerfeld zur Steuerung des Messablaufs der Messeinheit (18) aufweist.

7. Testgerät nach einem der Ansprüche 1 bis 6, **dadurch gekennzeichnet, dass** Start- und Stopp-Signale durch das Steuerfeld auslösbar sind.

8. Testgerät nach einem der Ansprüche 1 bis 7, **dadurch gekennzeichnet, dass** der Funktionsabschnitt (40) als Multifunktionsfeld für eine Kombination von mehreren Gerätehilfsfunktionen, insbesondere für eine Kalibration der Messeinheit (18) und Steuerung des Messablaufs ausgebildet ist.

9. Testgerät nach einem der Ansprüche 1 bis 8, **dadurch gekennzeichnet, dass** in Bandlaufrichtung Funktionsabschnitte (40) abwechselnd zu den Testabschnitten (38) auf derselben Bandseite angeordnet sind.

10. Testgerät nach einem der Ansprüche 1 bis 9, **dadurch gekennzeichnet, dass** mittels der an einem Funktionsabschnitt (40) kalibrierten Messeinheit (18) ein Leerwert an einem unbenutzten Testabschnitt (38) kontrollierbar ist.

11. Testgerät nach einem der Ansprüche 1 bis 10, **dadurch gekennzeichnet, dass** das Testband (16) von einer Vorratsspule (26) in dem Vorratsraum (24) abwickelbar, über Führungsmittel (19) im Bereich der Aufnahmeposition (20) führbar und auf eine Aufwickelspule (30) in dem Abfallraum (28) aufwicketbar ist.

12. Testgerät nach einem der Ansprüche 1 bis 11, **dadurch gekennzeichnet, dass** die Testabschnitte (38) durch auf einem kontinuierlichen Trägerband (36) abschnittsweise aufgebrachte Reagenzfelder, insbesondere durch eine Reagenzbeschichtung gebildet sind.

13. Testgerät nach einem der Ansprüche 1 bis 12, **dadurch gekennzeichnet, dass** das Testband (16) in einer Bandkassette (14) in ein Gehäuse (12) eines tragbaren Geräts (10) einsetzbar ist.

14. Bandkassette für ein Testgerät (10) nach einem der vorhergehenden Ansprüche, mit einem unter Bandvorlauf aus einem Vorratsraum (24) in einen Abfallraum (28) transportierbaren Testband (16), welches eine Vielzahl von an einer Aufnahmeposition (20) mit Körpedlüssigkeil beaufschlagbaren Testabschnitten (38) aufweist, wobei die Testabschnitte (38) durch Reagenzfelder gebildet sind, die auf einen inhaltsstoff der Körperflüssigkeit durch eine Farbänderung ansprechen, und wobei das Testband (16) mehrere durch Bandvorlauf in eine Funktionsstellung bringbare Funktionsabschnitte (40) für eine Zusatzfunktion zusätzlich zu den Testabschnitten (38) aufweist, **dadurch gekennzeichnet, dass** abwechselnd zu den Testabschnitten Funktionsabschnitte (40) angeordnet sind und ein weißes, schwarzes, graues, farbiges oder transparentes Kalibrierfeld zur Kalibration der Messeinheit (18) und/oder ein Steuerfeld zur Steuerung des Messablaufs der Messeinheit (18) aufweisen.

## Claims

1. Test instrument for analyzing body fluids such as blood or urine comprising a test tape (16) which is preferably in a tape cassette (14) and can be transported from a storage space (24) into a waste space (28) by advancing the tape, said test tape having a plurality of test sections to which body fluid can be applied at a receiving position (20), wherein the test sections (38) are formed by reagent fields which respond to a constituent of the body fluid by a colour change, and comprising an optical measuring unit (18) for detecting the constituent of the body fluid on an active test section (38), wherein the test tape (16) has, in addition to the test sections, a plurality of functional sections (40) for an auxiliary instrument function which can be brought into a functional position by advancing the tape, **characterized in that** functional sections (40) are arranged alternating to the test sections and have a white, black, grey, coloured or transparent calibration field for calibration of the measuring unit (18) and/or a control field for controlling the measuring process of the measuring unit (18).

2. Test instrument according to claim 1, **characterized in that** the functional section (40) interacts with the measuring unit (18) in the functional position.

3. Test instrument according to claim 1 or 2, **characterized in that** the calibration field has a defined optical property for a calibration of an optical measuring unit (18).

4. Test instrument according to one of the claims 1 to 3, **characterized in that** a transparent region of a carrier foil (36) of the test tape (16) is formed as a functional section for a dark calibration of the measuring unit (18).

5. Test instrument according to one of the claims 1 to 4, **characterized by** a comparator (42) for a comparison of a measuring value that has been detected at an unused test section (38) or at a functional section (40) with a set value which has been preferably stored in or supplied to the instrument.

6. Test instrument according to one of the claims 1 to 5, **characterized in that** the functional section (40) has a control field formed by a metal foil for controlling the measuring process of the measuring unit (18).

7. Test instrument according to one of the claims 1 to 6, **characterized in that** start and stop signals can be released by means of the control field.

8. Test instrument according to one of the claims 1 to 7, **characterized in that** the functional section (40) is designed as a multifunctional field for a combination of several auxiliary instrument functions, particularly for a calibration of the measuring unit (18) and control of the measuring process.

9. Test instrument according to one of the claims 1 to 8, **characterized in that** in tape advance direction, functional sections (40) are arranged alternately to the test sections (38) on the same side of the tape.

10. Test instrument according to one of the claims 1 to 9, **characterized in that** a blank value of an unused test section (38) can be controlled by means of the measuring unit being calibrated at a functional section (40).

11. Test instrument according to one of the claims 1 to 10, **characterized in that** the test tape (16) can be reeled off from a supply spool (26) in the storage space (24), guided by guiding means (19) in the area of the receiving position (20) and reeled onto a take-up spool (30) in the waste space (28).

12. Test instrument according to one of the claims 1 to 11, **characterized in that** the test sections (38) are formed by reagent fields, in particular by a reagent coating, that are applied in sections to a continuous carrier tape (36).

13. Test instrument according to one of the claims 1 to 12, **characterized in that** the test tape (16) in a tape cassette (14) can be inserted into a housing (12) of a portable instrument (10).

14. Tape cassette for a test instrument (10) according to one of the previous claims comprising a test tape (16) that can be transported from a storage space (24) into a waste space (28) by advancing the tape, said test tape having a plurality of test sections (38) to which body fluid can be applied at a receiving position (20), wherein the test sections (38)) are formed by reagent fields which respond to a constituent of the body fluid by a colour change, and wherein the test tape (16) has, in addition to the test sections (38), a plurality of functional sections (40) for an auxiliary instrument function which can be brought into a functional position by advancing the tape, **characterized in that** functional sections (40) are arranged alternating to the test sections and have a white, black, grey, coloured or transparent calibration field for calibration of the measuring unit (18) and/or a control field for controlling the measuring process of the measuring unit (18).

## Revendications

1. Appareil de test pour analyser des liquides organiques tels que du sang ou de l'urine, comprenant une bande de test (16) pouvant être transportée, de préférence dans une cartouche de bande (14) avec avancement de bande, d'un compartiment de réserve (24) dans un compartiment à déchets (28), laquelle bande présente une pluralité de plages de test aptes à recevoir du liquide organique au niveau d'une position de réception (20), les plages de test (38) étant formées par des zones réactives qui réagissent à une substance présente dans le liquide organique par un changement de couleur, et un ensemble de mesure optique (18) destiné à détecter la substance présente dans le liquide organique sur une plage de test active (38), ladite bande de test (16) présentant en plus desdites plages de test plusieurs plages fonctionnelles (40) pour une fonction auxiliaire de l'appareil, qui peuvent être amenées par avancement de bande dans une position fonctionnelle, **caractérisé en ce que** des plages fonctionnelles (40) sont disposées en alternance avec les plages de test et présentent une zone de calibrage blanche, noire, grise, colorée ou transparente pour le calibrage de l'ensemble de mesure (18) et/ou une zone de commande pour la commande du processus de mesure de l'ensemble de mesure (18).

2. Appareil de test selon la revendication 1, **caractérisé en ce que** la plage fonctionnelle (40), lorsqu'elle est en position fonctionnelle, coopère avec l'ensemble de mesure (18).

3. Appareil de test selon la revendication 1 ou 2, **caractérisé en ce que** la zone de calibrage possède une caractéristique optique définie pour un calibrage d'un ensemble de mesure optique (18).

4. Appareil de test selon l'une des revendications 1 à 3, **caractérisé en ce qu'**une zone transparente d'une feuille de support (36) de la bande de test (16) est conçue sous forme de plage fonctionnelle pour le calibrage obscur de l'ensemble de mesure (18).

5. Appareil de test selon l'une des revendication 1 à 4, **caractérisé par** un comparateur (42) pour la comparaison d'une valeur de mesure relevée sur une plage de test (38) inutilisée ou une plage fonctionnelle (40) avec une valeur de consigne de préférence déposée côté appareil ou introduite dans l'appareil.

6. Appareil de test selon l'une des revendications 1 à 5, **caractérisé en ce que** la plage fonctionnelle (40) présente une zone de commande formée par une feuille métallique pour la commande du processus de mesure de l'ensemble de mesure (18).

7. Appareil de test selon l'une des revendications 1 à 6, **caractérisé en ce que** des signaux de marche/arrêt peuvent être déclenchés par la zone de commande.

8. Appareil de test selon l'une des revendications 1 à 7, **caractérisé en ce que** la plage fonctionnelle (40) est conçue sous forme de zone multifonction pour une combinaison de plusieurs fonctions auxiliaires de l'appareil, en particulier pour un calibrage de l'ensemble de mesure (18) et une commande du processus de mesure.

9. Appareil de test selon l'une des revendications 1 à 8, **caractérisé en ce que** dans le sens de défilement de la bande, les plages fonctionnelles (40) sont disposées en alternance avec les plages de test (38) sur le même côté de la bande.

10. Appareil de test selon l'une des revendications 1 à 9, **caractérisé en ce que** l'ensemble de mesure (18) calibré sur une plage fonctionnelle (40) permet de contrôler une valeur à blanc sur une plage de test (38) inutilisée.

11. Appareil de test selon l'une des revendications 1 à 10, **caractérisé en ce que** la bande de test (16) peut être déroulée d'une bobine de réserve (26) dans le compartiment de réserve (24), guidée par des moyens de guidage (19) au niveau de la position de réception (20) et enroulable sur une bobine d'enroulement (30) dans le compartiment à déchets (28).

12. Appareil de test selon l'une des revendications 1 à 11, **caractérisé en ce que** les plages de test (38) sont formées par des zones réactives déposées par plages sur une bande de support continue (36), en particulier par un revêtement réactif.

13. Appareil de test selon l'une des revendications 1 à 12, **caractérisé en ce que** la bande de test (16) logée dans une cartouche de bande (14) peut être mise en place dans un boîtier (12) d'un appareil portatif (10).

14. Cartouche de bande pour un appareil de test (10) selon l'une des revendications précédentes, comprenant une bande de test (16) pouvant être transportée par avancement de bande d'un compartiment de réserve (24) dans un compartiment à déchets (28), laquelle bande présente une pluralité de plages de test (38) aptes à recevoir du liquide organique au niveau d'une position de réception (20), les plages de test (38) étant formées par des zones réactives qui réagissent à une substance présente dans le liquide organique par un changement de couleur, et ladite bande de test (16) présentant en plus desdites plages de test (38) plusieurs plages fonctionnelles (40) pour une fonction auxiliaire, qui peuvent être amenées par avancement de bande dans une position fonctionnelle, **caractérisée en ce que** des plages fonctionnelles (40) sont disposées en alternance avec les plages de test et présentent une zone de calibrage blanche, noire, grise, colorée ou transparente pour le calibrage de l'ensemble de mesure (18) et/ou une zone de commande pour la commande du processus de mesure de l'ensemble de mesure (18).
